# EUROPÄISCHE PATENTSCHRIFT

(11) **EP 0 731 090 B1**
(45) Veröffentlichungstag und Bekanntmachung des Hinweises auf die Patenterteilung: **06.05.2004**
(21) Anmeldenummer: 96106083.7
(22) Anmeldetag: 04.02.1992
(51) Int. Cl.: C07D 207/16, C07D 207/08, C07D 207/09, C07D 487/04, C07D 209/54

(54) **Verfahren zur Herstellung von 3,(4)-substituierten Pyrrolidinen**
Process for the production of 3,(4)-substituted pyrrolidines
Procédé pour la production de pyrrolidines 3,(4)-substitués

(30) Priorität: 17.02.1991 DE 4104870
(43) Veröffentlichungstag der Anmeldung: 11.09.1996
(62) Teilanmeldung aus: 92101809.9
(73) Patentinhaber: Bayer MaterialScience AG, 51368 Leverkusen (DE)
(72) Erfinder: Weider, Richard, Dr., 51381 Leverkusen (DE); Scholz, Uwe, Dr., 51065 Köln (DE); Ruckes, Andreas, Dr., 51381 Leverkusen (DE)

(56) Entgegenhaltungen:
- EP-A- 0 376 462
- US-A- 3 544 590
- US-A- 4 563 484
- SYNTHESIS, 1980, Seiten 811-812, XP002017979 T. ASAI ET AL.: "New Methods and Reagents in Organic Synthesis; 7. alpha-Alkylation of Benzylamine under Phase-Transfer Catalyzed Conditions"
- HEL. CHIM. ACTA, Bd. 64, Nr. 7, 1981, Seiten 2203-2218, XP002017980 R. ACHINI: "215. Synthesis of Phenyl- and Benzyl-Substituted Pyrrolidones and of a Piperidine by Intramolecular C-Alkylation. Synthons for Tricyclic Skeletons"
- GAZZ. CHIM. ITAL., Bd. 115, Nr. 10, 1985, Seiten 569-571, XP002017981 M. FORTE ET AL.: "Synthesis of pyrrolizidines and 1-oxapyrrolizidines from proline"
- J. HETEROCYCLIC CHEMISTRY, Bd. 25, Nr. 6, 1988, Seiten 1665-1673, XP002017982 F. ORSINI ET AL.: "1,3-Dipolar Cycloadditions of Azomethine Ylides with Dipolarophiles. II. Synthesis of Pyrrolizidines"

## Beschreibung

Die Erfindung betrifft ein Verfahren zur Herstellung von 3- und/oder 4-substituierten Pyrrolidinen, die als Katalysatoren zur Herstellung von Produkten nach dem Palyisocyanatpolyadditionsverfahren dienen. Sie können als Ersatz oder in Kombination mit an sich bekannten Urethankatalysatoren z.B. 1,4-Diazobicyclo[2,2,2]octan (DABCO) zur Herstellung von starren oder flexiblen Polyurethanschäumen sowie zahlreichen anderen Polyurethan-Produkten eingesetzt werden.

Die Herstellung der erfindungsgemäßen Pyrrolidine erfolgt aus gut verfügbaren und preiswerten Rohstoffen wie Aminosäurederivaten, Ketonen oder Aldehyden und Olefinen in sehr einfacher Weise und ist in den wesentlichen Stufen z.B. in Bull. Soc. Chim. France, 1988, S. 579-583 oder in Bull. Chem. Soc. Japan, 60, S. 4.079-4.090 (1989) beschrieben. Das dort beschriebene Verfahren erfordert allerdings zur Erzielung guter Ausbeuten sehr geringe Konzentrationen (unter 0,1 mol/l, vorteilhaft ca. 0,04 mol/l (s. Bull. Soc. Chim. France, S. 581 r.oben)) und damit für eine wirtschaftliche Herstellung nicht mehr akzeptable Lösungsmittelmengen. Führt man das beschriebene Verfahren bei höheren Konzentrationen (z.B. 1 mol/l) durch, sinken die Ausbeuten an gewünschten Verbindungen drastisch auf unter 30% zugunsten unlöslicher, nicht destillierbarer, inaktiver Harze. Selbst bei einer höheren Verdünnung zwischen 0,1 und 0,2 mol/l bewegen sich die Ausbeuten nicht in einer wirtschaftlich vertretbaren Größenordnung. Eine Verwendung der substituierten Pyrrolidine in technischem Maßstab ist nach diesem Verfahren nicht möglich.

Es wurde nun gefunden, daß man auch bei wesentlich höheren und damit wirtschaftlichen Konzentrationen zu sehr hohen Ausbeuten gelangt, wenn man die Keton- oder Aldehydkomponente während des Reaktionsverlaufs langsam zudosiert. Das Fortschreiten der Reaktion und damit die Zugabegeschwindigkeit ist leicht durch Gaschromatographie oder durch Bestimmung der Menge des gebildeten Reaktionswassers festzustellen.

Gegenstand der Erfindung ist daher auch ein Verfahren zur Herstellung von in 3-und/oder 4-Stellung substituierten Pyrrolidinen der Formel (I) 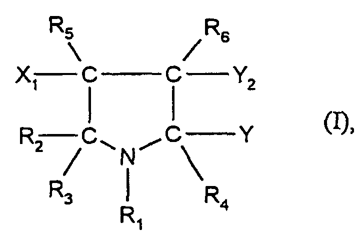 wobei
- R₁: Wasserstoff, eine C₁-C₁₂-Alkyl-, C₃-C₇-Cycloalkyl-, C₆-C₁₄-Aryl- oder - Arylalkyl-Gruppe, die gegebenenfalls jeweils N oder O enthalten können, oder gemeinsam mit R₄ eine gegebenenfalls substituierte C₁-C₅-Alkylengruppe oder eine C₁-C₆-Alkylengruppe, die zwei Pyrrolidinreste gemäß Formel (I) über das N-Atom verbindet;
- R₂, R₃: unabhängig voneinander Wasserstoff, eine C₁-C₁₂-Alkyl-, C₃-C₇-Cycloalkyl-, -Aryl- oder C₆-C₁₄-Arylalkylgruppe oder gemeinsam eine gegebenenfalls alkylsubstituierte C₂-C₉-Alkylengruppe;
- R₄: Wasserstoff, eine C₁-C₁₂-Alkyl-, C₃-C₇-Cycloalkyl-, C₆-C₁₄-Aryl- oder -Arylalkyl-Gruppe oder gemeinsam mit R₁ eine gegebenenfalls substituierte C₁-C₅-Alkylengruppe;
- R₅, R₆: Gruppen mit der gleichen Bedeutung wie Definition für R₂, R₃ oder gemeinsam einen Rest der Struktur: 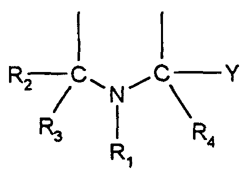
- X₁, X₂: unabhängig voneinander (d.h. gleich oder verschieden) Wasserstoff, Carbonsäuregruppen oder deren Ester, Amide, die gegebenenfalls O- oder N-Atome enthalten können, oder Nitril oder die Gruppen -CHR₇OH, -CH₂NR₈R₉, -CONR₈R₉ oder -NR₈R₉, wobei
R₇ Wasserstoff, eine C₁-C₁₂-Alkyl-, (gegebenenfalls alkylsubstituierte) -Cycloalkyl-, -Aryl-, -Arylalkylgruppe bedeutet und R₈, R₉ die Bedeutung wie Definition für R₂, R₃ besitzen;
- Y: Wasserstoff oder eine Carbonsäuregruppe oder deren funktionelles Derivat wie Ester, Amid, das gegebenenfalls O- oder N-Atome enthalten kann, oder Nitril oder die Gruppe -CHR₇OH oder -CH₂NR₈R₉;
darstellen, wobei mindestens einer der Reste X₁ oder X₂ von Wasserstoff verschieden sein muß und es sich im Fall X₁ ungleich X₂, um Gemische verschiedener Isomere bezüglich der Stellung der Substituenten X₁ und X₂ in der 3- oder 4-Position des Pyrrolidinrings, verschiedener Stereoisomere oder um isomerenreine Verbindungen handeln kann,
durch Umsetzung von α-Aminocarbonsäurederivaten der Formel (II) mit Ketonen oder Aldehyden der Formel (III) und aktivierten Olefinen der Formel (IV) zu Verbindungen der Formel (V) bei erhöhter Temperatur in einem inerten Lösungsmittel, vorzugsweise unter azeotroper Entfernung des Reaktionswassers nach Formelschema A, 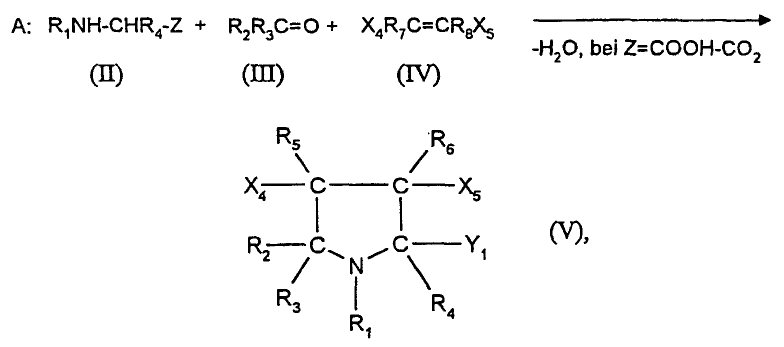 wobei
- R₁: Wasserstoff, eine C₁-C₁₂-Alkyl-, C₃-C₇-Cycloalkyl-, Aryl-, Arylalkyl-Gruppe, die N oder O enthalten können, oder gemeinsam mit R₄ eine gegebenenfalls substituierte C₁-C₅-Alkylengruppe oder eine C₁-C₆-Alkylengruppe, die zwei Reste gemäß Formel (V) bzw- (II) über das N-Atom verbindet;
- R₂, R₃: unabhängig voneinander Wasserstoff, eine C₁-C₁₂-Alkyl-, Cycloalkyl-, Aryl-, Arylalkylgruppe oder zusammen eine gegebenenfalls alkylsubstituierte C₁-C₉-Alkylengruppe;
- R₄: Wasserstoff, eine C₁-C₁₂-Alkyl-, C₃-C₇-Cycloalkyl-, Aryl-, Arylalkyl-Gruppe oder gemeinsam mit R₁ eine gegebenenfalls substituierte C₁-C₅-Alkylengruppe;
- R₅, R₆: Gruppen mit der Bedeutung aus Definition R₂, R₃ oder gemeinsam einen Rest der Struktur 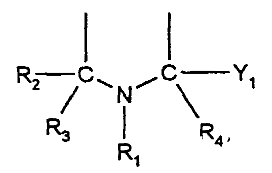
- R₇, R₈: Gruppen mit der Bedeutung aus Definition R₂, R₃ oder, wenn R₅ und R₆ gemeinsam die obengenannte Struktur annehmen, gemeinsam eine dritte Bindung;
- X₄, X₅: unabhängig voneinander Wasserstoff, Carbonsäuregruppen oder deren Ester oder Amide, die gegebenenfalls O- oder N-Atome enthalten können, oder Nitril oder die Gruppe NO₂;
- Z: eine Carbonsäuregruppe oder deren funktionelles Derivat wie Ester, Amid, die gegebenenfalls O- oder N-Atomen enthalten kann, oder Nitril; bedeuten,
und
- Y₁: identisch mit Z ist oder Wasserstoff bedeutet, wenn Z eine Carbonsäuregruppe darstellt,
das dadurch gekennzeichnet ist, daß das Keton oder der Aldehyd kontinuierlich oder portionsweise gerade so schnell zudosiert wird, wie die Reaktion fortschreitet und anschließend gegebenenfalls nach Isolierung des Produktes (V) gegebenenfalls in an sich bekannter Weise X₄, X₅ und/oder Y₁ der Verbindung (V) in den erweiterten Bedeutungsumfang von X₁, X₂ und/oder Y der Formel (I) transformiert wird.

Bevorzugte Pyrrolidin-Verbindungen sind dabei solche der allgemeinen Formel (Ia) 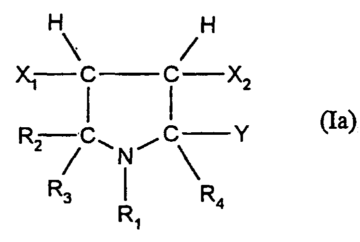 wobei R₁ bis R₄ die Bedeutung aus Formel (I) haben und
- X₁, X₂: unabhängig voneinander Wasserstoff, eine Carbonsäureester-, -amid-, die gegebenenfalls O- oder N-Atome enthalten kann, oder Nitrilgruppe oder die Gruppen -CH₂OH oder -CH₂NH₂, und
- Y: Wasserstoff oder die Gruppe -CH₂OH oder -CH₂NH₂;
bedeuten.

Besonders bevorzugt werden solche Pyrrolidine der allgemeinen Formel (Ib) 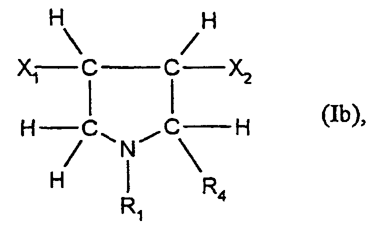 wobei X₁ und X₂ die Bedeutung gemäß Formel (Ia) haben und
- R₁: eine C₁-C₁₂-Alkyl-, C₅-C67-Cycloalkyl-, Aryl-, Arylalkyl-Gruppe, oder zusammen mit R₄ eine gegebenenfalls substituierte C₃-C₅-Alkylengruppe;
- R₄: Wasserstoff oder zusammen mit R₁ eine gegebenenfalls substituierte C₃-C₅-Alkylengruppe
bedeuten.

Weiterhin bevorzugt sind neue Verbindungen der allgemeinen Formel (Ic) 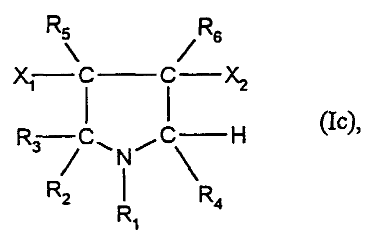 in der
- R₁, R₂, R₃: unabhängig voneinander Wasserstoff, eine C₁-C₁₂-Alkyl, Cycloalkyl-, Acyl-, oder Aralkylgruppe bedeuten,
- X₁: und mindestens einer der ResteR₅ oder R₆ Wasserstoff ist,
- X₂: eine Gruppe -CH₂NH₂, -CH₂OH, -CN oder -CONR₈R₉ bedeutet,
- R₄, (R₅ bzw. R₆), R₈ und R₉: die Bedeutung der Formel I haben, und
wenn R₁ für Wasserstoff oder Methyl und gegebenenfalls R₉ für Methyl steht, mindestens einer der Reste R₂, R₃, R₄ und ( R₅ bzw. R₆) nicht für Wasserstoff steht;
und deren Isomere und Isomerengemische bezüglich Vertauschung von X₁ und X₂.

Weiterhin bevorzugt sind neue Verbindungen der allgemeinen Formel (Id): 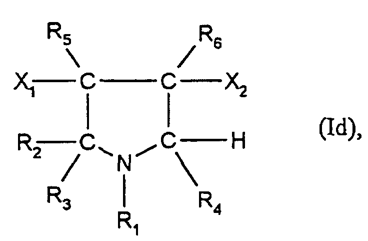 wobei R₂, R₃, R₅, R₆, X₁, X₂ die Bedeutung aus Formel (I) haben; und
- R₁: gemeinsam mit R₄ eine gegebenenfalls substituierte C₂-C₄-Alkylengruppe darstellen, und wobei
mindestens einer der Reste R₂, R₃, R₅ oder R₆ nicht für Wasserstoff steht, wenn einer der Reste X für Wasserstoff oder der andere Rest X für die Gruppe COOCH₃ oder COOC₂H₅ stehen.

Durch die erfindungsgemäße allmähliche Zugabe werden Konzentrationen des Endproduktes in der Reaktionslösung von mindestens 0,2 mol/l erreicht. Besonders bevorzugt liegt die Konzentration des Endproduktes zwischen 0,5 und 2 mol/l.

α-Aminocarbonsäurederivate sind z.B. α-Aminosäuren wie Glycin, N-Methylglycin (Sarkosin), N-Phenylglycin, N-Benzylglycin, N-Triphenylmethylglycin, Alanin, N-Methylalanin, N-Phenylalanin, Lysin, N-Methyllysin, Methionin, N-Methyl--Methionin, 2-Aminomalonsäure, 2-Methylaminobernsteinsäure, cyclische Aminosäuren wie Prolin oder Piperidin-2-carbonsäure, die Methyl- oder Ethylester der genannten Aminosäuren oder α-Aminonitrile wie z.B. 2-Aminoacetonitril, 2-N-Methylaminoacetonitril. Besonders bevorzugt sind N-Methylglycin und Prolin.

Ketone sind z.B. Aceton, Methyl-ethylketon, 2-Pentanon, 3-Pentanon, Methyl-isopropyl-keton, 4-Heptanon, Methyl-nonyl-keton, Dinonylketon, Cyclohexanon, Methylcyclohexanon, Cyclopentanon, Acetophenon, 4-Chloracetophenon, oder Benzophenon. Aldehyde sind z.B. Formaldehyd oder dessen Derivate wie Trioxan, Paraformaldehyd oder Dimethoxymethan, Acetaldehyd, Butyraldehyd, Isobutyraldehyd, Pivalinaldehyd, Önanthaldehyd, 2-Ethylhexanal, Benzaldehyd, 3-Methylbenzaldehyd, Propiophenon oder 3-Phenyl-propionaldehyd. Besonders bevorzugt sind Formaldehyd und Paraformaldehyd.

Unter aktivierten Olefinen sind solche zu verstehen, die in Konjugation zur Doppel- bzw. Dreifachbindung mindestens eine aktivierende Gruppe X₄ oder X₅ mit dem oben angegebenen Bedeutungsumfang besitzen. Hierzu gehören z.B. Acrylsäure, Methacrylsäure, Crotonsäure, 3-Methyl-crotonsäure, Sorbinsäure, Fumarsäure, Maleinsäure, Itaconsäure, Zimtsäure, Acetylendicarbonsäure sowie die Methyl-, Ethyl- oder Butylester der genannten Säuren, Acrylnitril Methacrylnitril oder Acetylendicarbonsäuredinitril. Setzt man statt der Olefine VI Acetylene ein (R₁₁, R₁₂ = Bindung), gelangt man zu den Bicyclo-3,7-diazaoctanderivaten. Besonders bevorzugt sind Acrylsäuremethyl-, -ethyl- und -butylester, Maleinsäuremethylund -ethylester und Acrylnitril.

Als inerte Lösungsmittel sind solche bevorzugt, deren Siedepunkt über 70°C liegt. Die Ausgangsstoffe für die Reaktion können in den Lösungsmitteln gelöst oder in suspendierter Form vorliegen. Gegebenenfalls kann auch auf eine Lösungsmittel verzichtet werden und statt dessen ein Überschuß der Olefinkomponente verwendet werden. Nahezu alle technisch verwendeten Lösemittel mit Ausnahme von Ketonen und Wasser sind gegenüber der Reaktion inert, es seien daher nur einige Beispiele genannt: Petrolether, Benzin, Benzol, Toluol, Xylole, Chlorbenzol, Dichlorbenzole, Anisol, Ethanol, Isopropanol, Butanol, Dioxan, Dimethylformamid oder Dimethylacetamid oder Gemische aus den genannten Lösemitteln: Besonders bevorzugt sind Toluol und Dimethylformamid oder Gemische daraus.

In einer bevorzugten Ausführungsform wird das Reaktionswasser als Azeotrop entfernt, es kann aber auch, wie z.B. bei Verwendung von Dimethylformamid, in dem Reaktionsgemisch verbleiben oder in anderer Weise, z.B. durch bekannte inerte, wasserentziehende Mittel z.B. Molekularsiebe oder destillativ ohne Verwendung eines Azeotrops entfernt werden.

Viele der Produkte, die nach Schema A erhältlich sind, sind bereits hochwirksame Katalysatoren, deren Wirkung aber noch erhöht werden kann, wenn man die funktionellen Gruppen X₄, X₅ und Y₁ durch einfache, jedem Fachmann bekannte Transformationen wie z.B. Aminolyse mit primären oder sekundären, gegebenenfalls N- oder O-Atome enthaltenden Aminen, Umesterung mit gegebenenfalls N-oder O-Atomen enthaltenden Alkoholen oder Reduktion in die Gruppen mit dem überschüssigen Bedeutungsumfang von X₁, X₂ und Y der Formel (I) umwandelt. Speziell die einbaufähigen Vertreter mit isocyanatreaktiven Gruppen werden auf diese Art erhalten.

Eine konkrete Synthesefolge soll an dem folgenden Formelschema stellvertretend dargestellt werden und das Verfahren verdeutlichen:

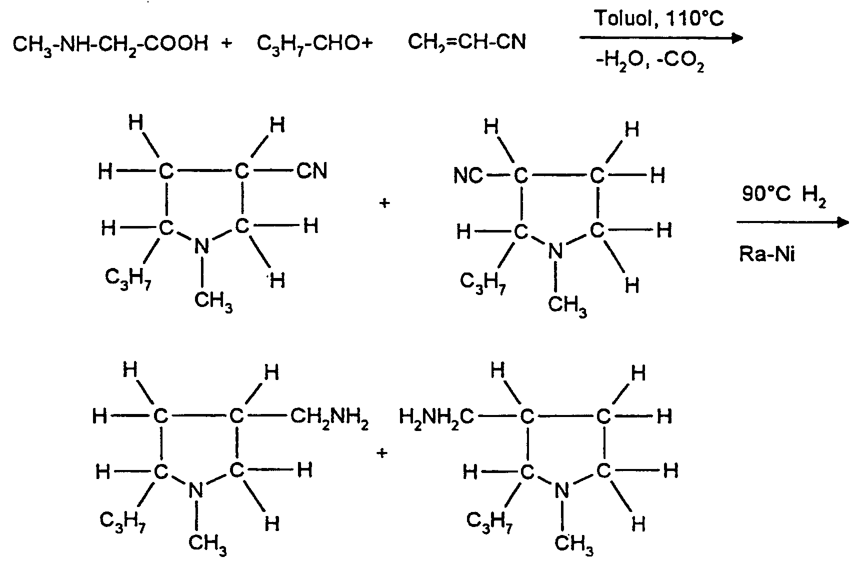

Bevorzugte erfindungsgemäße Pyrrolidine sind z.B.:
3-Hydroxymethyl-N-methyl-pyrrolidin, 3,4-Bis-hydroxymethyl-N-methyl-pyrrolidin, 3-Aminomethyl-N-methyl-pyrrolidin, N-Methyl-pyrrolidin-3-carbonsäure-N-(3-dimethylaminopropyl)-amid, 1-Azabicyclo[3,3,0]-octan-3-carbonsäurebutylester, 1-Azabicyclo[3,3,0]-octan-4-carbonsäurebutylester, 1-Azabicyclo[4,3,0]-nonan-3-carbonsäurebutylester, 1-Azabicyclo[4,3,0]-nonan-4-carbonsäurebutylester, 3-Cyano-1-azabicyclo[3,3,0]-octan, 4-Cyano-1-azäbicyclo[3,3,0]-octan, 3-Aminomethyl--1-azabicyclo[3,3,0]-octan und 4-Aminomethyl-1-azabicyclo[3,3,0]-octan.

Besonders bevorzugt sind 3-Hydroxymethyl-N-methyl-pyrrolidin, 3,4-Bis-hydroxymethyl-N-methyl-pyrrolidin, 3-Aminomethyl-N-methyl-pyrrolidin, N-Methyl-pyrrolidin-3-carbonsäure-N-(3-dimethylaminopropyl)-amid, 1-Azabicyclo[3,3,0]-octan-3-carbonsäurebutylester, 1-Azabicyclo[3,3,0]-octan-4-carbonsäurebutylester,3-Aminomethyl-1-azabicyclo[3,3,0]-octan und 4-Aminomethyl-1-azabicyclo[3,3,0]-octan.

Die erfindungsgemäßen Pyrrolidine sind farblose bis leicht gelbliche Verbindungen, wobei die bevorzugten Typen flüssig sind. Sie sind löslich in organischen Lösungsmitteln und löslich oder dispergierbar in Wasser und sind wertvolle Katalysatoren.

Die nachfolgenden Beispiele erläutern die Erfindung weiter, ohne sie zu beschränken. Teile und Verhältnisse sind in allen Fällen als Gewichtsteile gemeint.

### Beispiel

### Beispiel 1

In diesem Beispiel wird das Verfahren zur Herstellung des 3-Cyano-N-methylpyrrolidins und des 3-Aminomethyl-N-methylpyrrolidins beschrieben:
a) Vergleichsbeispiel (nicht erfindungsgemäßes Verfahren):
   In einem 4 l-Dreihalskolben, ausgerüstet mit Rührer, Rückflußkühler und einem ca. 70 ml fassenden Wasserabscheider werden 300 g N-Methylglycin, 300 g Acrylnitril, 105 g Paraformaldehyd und 3 l Toluol vorgelegt und bei einer Badtemperatur von 120 bis 140°C zum kräftigen Rückfluß erhitzt, bis die Wasserentwicklung abgeschlossen ist. Am Ende der Reaktion erhält man eine braungelbe Lösung, aus der sich ein dunkelbraunes Harz abgeschieden hat. Das Lösemittel wird abdestilliert und der Rückstand im Vakuum fraktioniert. Man erhält 96 g (26 % der Theorie) 3-Cyano-N-methylpyrrolidin.
b) Vergleichsbeispiel (nicht erfindungsgemäßes Verfahren):
   In einem 4 l-Dreihalskolben, ausgerüstet mit Rührer, Rückflußkühler und einem ca. 70 ml fassenden Wasserabscheider werden 50 g N-Methylglycin, 60 g Acrylnitril, 25 g Paraformaldehyd und 3 l Toluol vorgelegt und bei einer Badtemperatur von 120 bis 140°C zum kräftigen Rückfluß erhitzt, bis die Wasserentwicklung abgeschlossen ist. Am Ende der Reaktion erhält man eine braungelbe Lösung. Das Lösemittel wird abdestilliert und der Rückstand im Vakuum fraktioniert. Man erhält 32 g (51 % der Theorie) 3-Cyano-N-methylpyrrolidin.
c) Erfindungsgemäßes Verfahren:
   In einem 4 l-Dreihalskolben, ausgerüstet mit Rührer, Rückflußkühler, Dosiertrichter und einem ca. 70 ml fassenden Wasserabscheider werden 300 g N-Methylglycin, 300 g Acrylnitril und 3 l Toluol vorgelegt und bei einer Badtemperatur von 120 bis 140°C zum kräftigen Rückfluß erhitzt. Dann werden in Portionen von 3 g insgesamt 105 g Paraformaldehyd zudosiert, wobei jeweils solange mit der Zugabe der nächsten Portion gewartet wird, bis die Wasserentwicklung abgeschlossen ist. Am Ende der Reaktion erhält man eine homogene, leicht gelbliche Lösung. Das Lösemittel wird abdestilliert und der Rückstand im Vakuum fraktioniert. Man erhält 315 g (85 % der Theorie) 3-Cyano-N-methylpyrrolidin (Kp.: 83-85°C, 22 mmHg).
   Die anschließende Reduktion zum 3-Aminomethyl-N-methylpyrrolidin erfolgt nach bekanntem Verfahren:
   Man löst die Cyanverbindung im gleichen Volumen Methanol, füllt die Lösung in einen 2 l-Autoklaven, gibt 20 g Raney-Kobalt dazu und drückt 130 g Ammoniak auf. Dann wird bei einem Wasserstoffdruck von 90 bis 100 bar bei 90°C hydriert (ca. 3 Stunden). Die entspannte Lösung wird filtriert, eingedampft und der Rückstand im Vakuum fraktioniert. Man erhält 310 g (95 % der Theorie) 3-Aminomethyl-N-methylpyrrolidin (Kp.: 61°C, 22 mbar).

### Beispiele 2 bis 9

Nach dem in Beispiel 1 c) beschriebenen Verfahren werden unter den gleichen Bedingungen die im Anhang formelmäßig angegebenen Verbindungen hergestellt. Tabelle 1 gibt die Ausgangsstoffe sowie die erzielten Ausbeuten (% der Theorie, bezogen auf Aminosäurederivat) nach destillativer Reinigung an:

**Tabelle 1**

| Beispiel | Aminosäurederivat | Keton/Aldehyd | Olefin | Ausbeute |
|---|---|---|---|---|
| 2 | Methylglycin | Butyraldehyd | Acrylnitril | 87 % |
| 3 | Methylglycin | Aceton | Methylacrylat | 79 % |
| 4 | Methylglycin | Paraformaldehyd | Butylacrylat | 89 % |
| 5 | Methylglycin | Paraformaldehyd | Methylacrylat | 81 % |
| 6 | Methylglycin | Paraformaldehyd | Diethylmaleat | 85 % |
| 7 | DL-Prolin | Paraformaldehyd | Butylacrylat | 95 % |
| 8 | DL-Prolin | Paraformaldehyd | Acrylnitril | 92 % |
| 9 | DL-Prolin | Paraformaldehyd | Diethylmaleat | 90 % |
| Physikalische Daten der Verbindungen 2 bis 9: 2: Kp.: 103-110°C (20 mbar); 3: (2 Isomere) Kp.: 75-78°C (11 mbar); 4: Kp.: 105°C (12 mbar); 5: Kp.: 71°C (12 mbar); 6: (2 Isomere) Kp.: 140-145°C (11 mbar); 7: (4 Isomere) Kp.: 140-147°C (12 mbar); 8: (4 Isomere) Kp.: 115-120°C (22 mbar); 9: (6 Isomere) Kp.: 175-185°C (10 mbar). | | | | |

### Beispiele 10 bis 14

Die Verbindungen 2 und 8 werden wie in Beispiel 1a) zu den Verbindungen 10 und 11 hydriert, die Verbindungen 5 und 6 werden durch Reduktion mit Natriumborhydrid nach bekanntem Verfahren zu den Verbindungen 12 und 13 reduziert. Die Verbindung 5 wird durch Aminolyse mit 1-Amino-3-dimethylamino-propan nach bekanntem Verfahren zur Verbindung 14 umgesetzt. Die Strukturen sind dem Anhang zu entnehmen.

### ANHANG

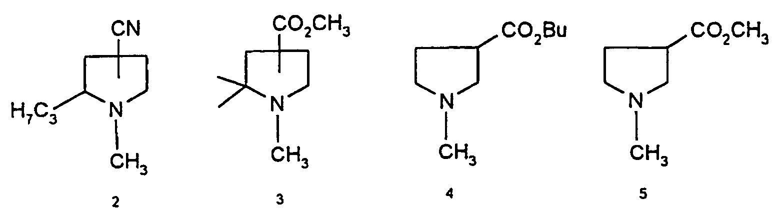

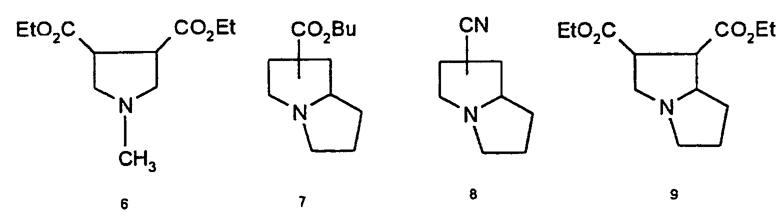

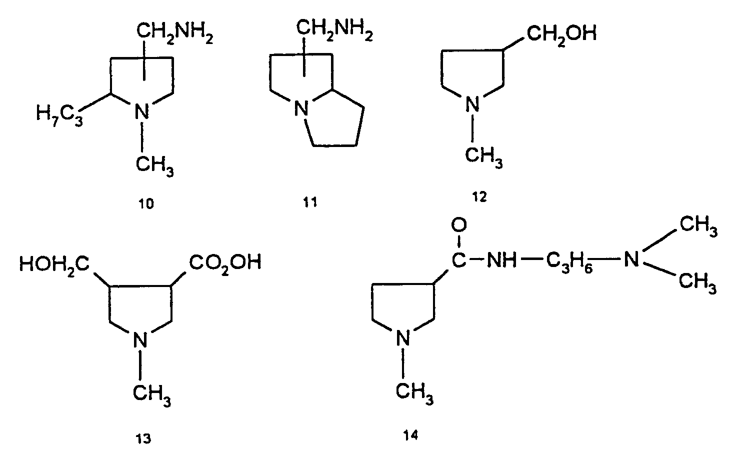

## Patentansprüche

1. Verfahren zur Herstellung von in 3- und/oder 4-Stellung substituierten Pyrrolidinen der Formel (I) 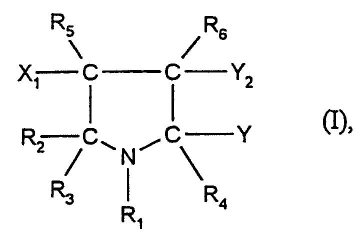 wobei
R₁ Wasserstoff, eine C₁-C₁₂-Alkyl-, C₃-C₇-Cycloalkyl-, C₆-C₁₄-Aryl- oder -Arylalkyl-Gruppe, die gegebenenfalls jeweils N oder O enthalten können, oder gemeinsam mit R₄ eine gegebenenfalls substituierte C₁-C₅-Alkylengruppe oder eine C₁-C₆-Alkylengruppe, die zwei Pyrrolidinreste gemäß Formel (I) über das N-Atom verbindet;
R₂, R₃ unabhängig voneinander Wasserstoff, eine C₁-C₁₂-Alkyl-, (gegebenenfalls alkylsubstituierte) C₃-C₇-Cycloalkyl-, -Aryl- oder C₆-C₁₄-Arylalkylgruppe oder gemeinsam eine gegebenenfalls alkylsubstituierte C₂-C₉-Alkylengruppe;
R₄ Wasserstoff, eine C₁-C₁₂-Alkyl-, (gegebenenfalls alkylsubstituierte) C₃-C₇-Cycloalkyl-, C₆-C₁₄-Aryl- oder -Arylalkyl-Gruppe oder gemeinsam mit R₁ eine gegebenenfalls substituierte C₁-C₅-Alkylengruppe;
R₅, R₆ Gruppen mit der gleichen Bedeutung wie Definition für R₂, R₃ oder gemeinsam einen Rest der Struktur: 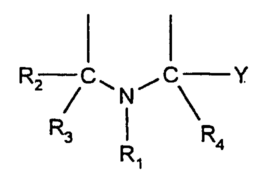
X₁, X₂ unabhängig voneinander (d.h. gleich oder verschieden) Wasserstoff, Carbonsäuregruppen oder deren Ester, Amide, die gegebenenfalls O- oder N-Atome enthalten können, oder Nitril oder die Gruppen -CHR₇OH, -CH₂NR₈R₉, -CONR₈R₉ oder -NR₈R₉, wobei
R₇ Wasserstoff, eine C₁-C₁₂-Alkyl-, (gegebenenfalls alkylsubstituierte) -Cycloalkyl-, -Aryl-, -Arylalkylgruppe bedeutet und R₈, R₉ die Bedeutung wie Definition für R₂, R₃ besitzen;
Y Wasserstoff oder eine Carbonsäuregruppe oder deren funktionelles Derivat wie Ester, Amid, das gegebenenfalls O- oder N-Atome enthalten kann, oder Nitril oder die Gruppe -CHR₇OH oder -CH₂NR₈R₉;
darstellen, wobei mindestens einer der Reste X₁ oder X₂ von Wasserstoff verschieden sein muß und es sich im Fall X₁ ungleich X₂, um Gemische verschiedener Isomere bezüglich der Stellung der Substituenten X₁ und X₂ in der 3- oder 4-Position des Pyrrolidinrings, verschiedener Stereoisomere oder um isomerenreine Verbindungen handeln kann,
durch Umsetzung von α-Aminocarbonsäurederivaten der Formel (II) mit Ketonen oder Aldehyden der Formel (III) und aktivierten Olefinen der Formel (IV) zu Verbindungen der Formel (V) bei erhöhter Temperatur in einem inerten Lösungsmittel, vorzugsweise unter azeotroper Entfernung des Reaktionswassers nach Formelschema A, 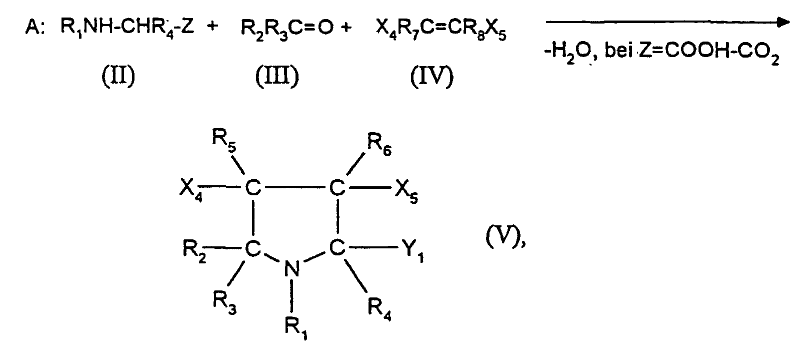 wobei
R₁ Wasserstoff, eine C₁-C₁₂-Alkyl-, C₃-C₇-Cycloalkyl-, Aryl-, Arylalkyl-Gruppe, die N oder O enthalten können, oder gemeinsam mit R₄ eine gegebenenfalls substituierte C₁-C₅-Alkylengruppe oder eine C₁-C₆-Alkylengruppe, die zwei Reste gemäß Formel (V) bzw- (II) über das N-Atom verbindet;
R₂, R₃ unabhängig voneinander Wasserstoff, eine C₁-C₁₂-Alkyl-, Cycloalkyl-, Aryl-, Arylalkylgruppe oder zusammen eine gegebenenfalls alkylsubstituierte C₁-C₉-Alkylengruppe;
R₄ Wasserstoff, eine C₁-C₁₂-Alkyl-, C₃-C₇-Cycloalkyl-, Aryl-, Arylalkyl-Gruppe oder gemeinsam mit R₁ eine gegebenenfalls substituierte C₁-C₅-Alkylengruppe;
R₅, R₆ Gruppen mit der Bedeutung aus Definition R₂, R₃ oder gemeinsam einen Rest der Struktur 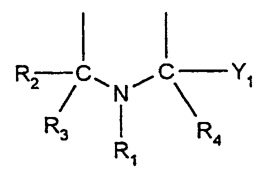
R₇, R₈ Gruppen mit der Bedeutung aus Definition R₂, R₃ oder, wenn R₅ und R₆ gemeinsam die obengenannte Struktur annehmen, gemeinsam eine dritte Bindung;
X₄, X₅ unabhängig voneinander Wasserstoff, Carbonsäuregruppen oder deren Ester oder Amide, die gegebenenfalls O- oder N-Atome enthalten können, oder Nitril oder die Gruppe NO₂;
Z eine Carbonsäuregruppe oder deren funktionelles Derivat wie Ester, Amid, die gegebenenfalls O- oder N-Atomen enthalten kann, oder Nitril; bedeuten,
und
Y₁ identisch mit Z ist oder Wasserstoff bedeutet, wenn Z eine Carbonsäuregruppe darstellt,
**dadurch gekennzeichnet, daß** das Keton oder der Aldehyd kontinuierlich oder portionsweise gerade so schnell zudosiert wird, wie die Reaktion fortschreitet bis die Konzentration des Produktes (V) größer alls 0,2 mol/l beträgt und anschließend gegebenenfalls nach Isolierung des Produktes (V) in an sich bekannter Weise X₄, X₅ und/oder Y₁ der Verbindung (V) in den erweiterten Bedeutungsumfang von X₁, X₂ und/oder Y der Formel (I) überführt.

## Claims

1. Process for the production of pyrrolidines substituted in 3 and/or 4 position of formula (I) 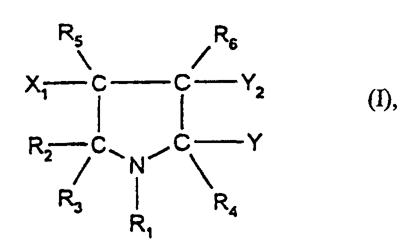 wherein
R₁ represents hydrogen, a C₁-C₁₂ alkyl, C₃-C₇ cycloalkyl, C₆-C₁₄ aryl or arylalkyl group, which may optionally each contain N or O, or, together with R₄, represents an optionally substituted C₁-C₅ alkylene group or a C₁-C₆ alkylene group connecting two pyrrolidine groups according to formula (I) via the N atom;
R₂ and R₃, independently of one another, represent hydrogen, a C₁-C₁₂ alkyl, (optionally alkyl-substituted) C₃-C₇ cycloalkyl, aryl or C₆-C₁₄ arylalkyl group or together represent an optionally alkyl-substituted C₂-C₉ alkylene group;
R₄ represents hydrogen, a C₁-C₁₂ alkyl, (optionally alkyl-substituted) C₃-C₇ cycloalkyl, C₆-C₁₄ aryl or arylalkyl group or, together with R₁, represents an optionally substituted C₁-C₅ alkylene group;
R₅ and R₆ represent groups with the same meaning as the definition of R₂ and R₃ or together represent a group having the following structure: 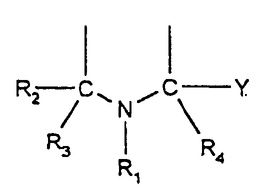
X₁ and X₂, independently of one another (i.e. being the same or different), represent hydrogen, carboxylic acid groups or their esters or amides, which may optionally contain O or N atoms, or nitrile or the groups -CHR₇OH, -CH₂NR₈R₉, -CONR₈R₉ or -NR₈R₉, wherein
R₇ signifies hydrogen, a C₁-C₁₂ alkyl, (optionally alkyl-substituted) cycloalkyl, aryl, arylalkyl group and R₈ and R₉ have the meaning as in the definition of R₂ and R₃;
Y represents hydrogen or a carboxylic acid group or the functional derivative thereof, such as ester or amide, which may optionally contain O or N atoms, or nitrile or the group -CHR₇OH or -CH₂NR₈R₉;
wherein at least one of the groups X₁ or X₂ must be other than hydrogen and, when X₁ and X₂ are not identical, the compounds may be mixtures of different isomers in terms of the position of the substituents X₁ and X₂ in the 3 or 4 position of the pyrrolidine ring, different stereoisomers or isomerically pure compounds,
by reacting α-aminocarboxylic acid derivatives of formula (II) with ketones or aldehydes of formula (III) and activated olefins of formula (IV) to form compounds of formula (V) at an elevated temperature in an inert solvent, preferably with the azeotropic removal of the water of reaction in accordance with formula diagram A, 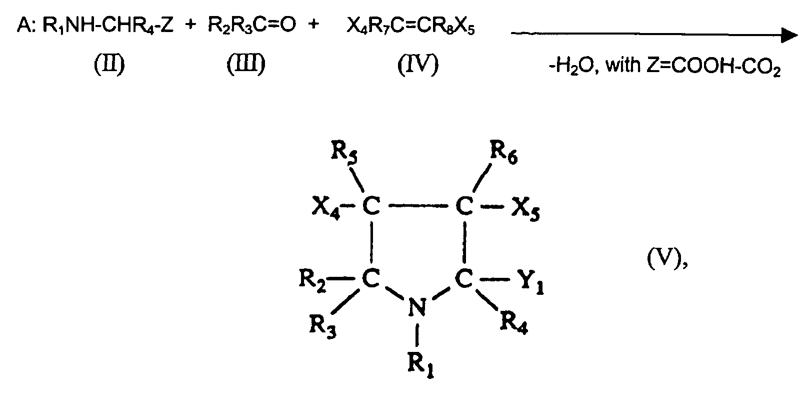 wherein
R₁ signifies hydrogen, a C₁-C₁₂ alkyl, C₃-C₇ cycloalkyl, aryl or arylalkyl group, which may contain N or O, or, together with R₄, signifies an optionally substituted C₁-C₅ alkylene group or a C₁-C₆ alkylene group connecting two groups according to formula (V) or (II) via the N atom;
R₂ and R₃, independently of one another, signify hydrogen, a C₁-C₁₂ alkyl, cycloalkyl, aryl or arylalkyl group or together signify an optionally alkyl-substituted C₁-C₉ alkylene group;
R₄ signifies hydrogen, a C₁-C₁₂ alkyl, C₃-C₇ cycloalkyl, aryl or arylalkyl group or, together with R₁, signifies an optionally substituted C₁-C₅ alkylene group;
R₅ and R₆ signify groups with the meaning from the definition of R₂ and R₃ or together signify a group having the following structure: 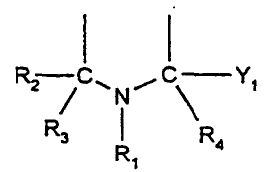
R₇ and R₈ signify groups with the meaning from the definition of R₂ and R₃ or, if R₅ and R₆ together take on the above structure, they together signify a third bond;
X₄ and X₅, independently of one another, signify hydrogen, carboxylic acid groups or their esters or amides, which may optionally contain O or N atoms, or nitrile or the group NO₂;
Z signifies a carboxylic acid group or its functional derivative, such as ester or amide, which may optionally contain O or N atoms, or nitrile;
and
Y₁ is identical to Z or signifies hydrogen if Z represents a carboxylic acid group,
**characterised in that** the ketone or aldehyde is metered in, continuously or in portions, at precisely the rate at which the reaction progresses, until the concentration of the product (V) is greater than 0.2 mol/l and then, optionally after isolating the product (V), X₄, X₅ and/or Y₁ of the compound (V) is converted, in a manner that is known *per se*, into the extended scope of meaning of X₁, X₂ and/or Y of formula (I).

## Revendications

1. Procédé de préparation de pyrrolidines substituées en 3 et/ou 4 de formule (I): 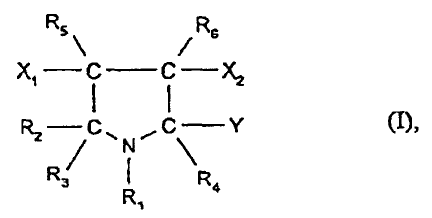 dans laquelle
R₁ représente un atome d'hydrogène, un groupe alkyle en C₁-C₁₂, cycloalkyle en C₃-C₇, aryle ou arylalkyle en C₆-C₁₄, pouvant chacun éventuellement contenir N ou O, ou, en combinaison avec R₄, un groupe alkylène en C₁-C₅ éventuellement substitué ou un groupe alkylène en C₁-C₆ qui relie deux restes pyrrolidine de formule (I) par l'intermédiaire de l'atome d'azote;
R₂, R₃ représentent indépendamment l'un de l'autre un atome d'hydrogène ou un groupe alkyle en C₁-C₁₂, cycloalkyle en C₃-C₇, aryle ou arylalkyle en C₆-C₁₄ (éventuellement substitué par alkyle), ou forment ensemble un groupe alkylène en C₂-C₉ éventuellement substitué par alkyle;
R₄ représente un atome d'hydrogène ou un groupe alkyle en C₁-C₁₂, cycloalkyle en C₃-C₇, aryle ou arylalkyle en C₆-C₁₄ (éventuellement substitué par alkyle), ou forme en combinaison avec R₁ un groupe alkylène en C₁-C₅ éventuellement substitué;
R₅, R₆ représentent des groupes ayant la même signification que la définition de R₂, R₃, ou forment ensemble un reste de structure 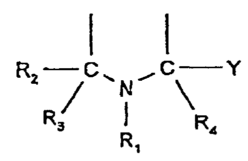
X₁, X₂ représentent, indépendamment l'un de l'autre (c'est-à-dire qu'ils sont identiques ou différents), un atome d'hydrogène, un groupe acide carboxylique ou un de ses esters ou amides, pouvant éventuellement contenir des atomes de O ou N, un groupe nitrile ou un groupe -CHR₇OH, -CH₂NR₈R₉, -CONR₈R₉ ou - NR₈R₉, où
R₇ représente un atome d'hydrogène, un groupe alkyle en C₁-C₁₂, ou un groupe cycloalkyle, aryle ou arylalkyle (éventuellement substitué par alkyle), et
R₈, R₉ ont la signification donnée par la définition de R₂, R₃;
Y représente un atome d'hydrogène ou un groupe acide carboxylique ou un de ses dérivés fonctionnels comme un ester, un amide, pouvant éventuellement contenir des atomes de O ou N, un groupe nitrile ou un groupe -CHR₇OH ou - CH₂NR₈R₉;
au moins l'un des restes X₁ et X₂ devant être différent de l'hydrogène et, dans le cas où X₁ est différent de X₂, ces pyrrolidines peuvent être constituées de mélanges de différents isomères par rapport à la position des substituants X₁ et X₂ en position 3 ou 4 du cycle pyrrolidine, de mélanges de différents stéréoisomères ou de composés isomères purs,
par réaction de dérivés d'acides α-aminocarboxyliques de formule (II) avec des cétones ou des aldéhydes de formule (III) et des oléfines activées de formule (IV) donnant des composés de formule (V) à température élevée, dans un solvant inerte, de préférence avec distillation azéotropique de l'eau de réaction, selon le schéma réactionnel A 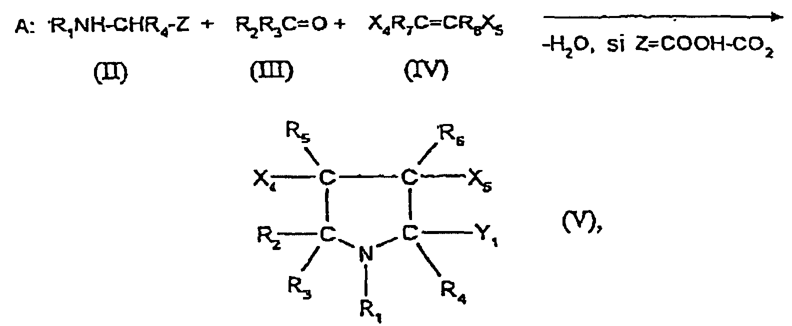 où
R₁ représente un atome d'hydrogène, un groupe alkyle en C₁-C₁₂, cycloalkyle en C₃-C₇, aryle ou arylalkyle, pouvant contenir N ou O, ou, en combinaison avec R₄, un groupe alkylène en C₁-C₅ éventuellement substitué ou un groupe alkylène en C₁-C₆ qui relie deux restes de formule (V) ou (II) par l'intermédiaire de l'atome d'azote;
R₂, R₃ représentent indépendamment l'un de l'autre un atome d'hydrogène ou un groupe alkyle en C₁-C₁₂, cycloalkyle, aryle ou arylalkyle, ou forment ensemble un groupe alkylène en C₁-C₉ éventuellement substitué par alkyle;
R₄ représente un atome d'hydrogène ou un groupe alkyle en C₁-C₁₂, cycloalkyle en C₃-C₇, aryle ou arylalkyle, ou forme en combinaison avec R₁ un groupe alkylène en C₁-C₅ éventuellement substitué;
R₅, R₆ représentent des groupes ayant la signification donnée par la définition de R₂, R₃, ou forment ensemble un reste de structure 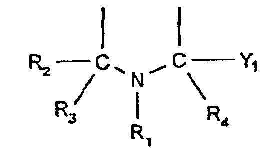
R₇, R₈ représentent des groupes ayant la signification donnée par la définition de R₂, R₃ ou, lorsque R₅ et R₆ forment ensemble la structure indiquée ci-dessus, R₇ et R₈ forment ensemble une troisième liaison,
X₄, X₅ représentent, indépendamment l'un de l'autre, un atome d'hydrogène, un groupe acide carboxylique ou un de ses esters ou amides, pouvant éventuellement contenir des atomes de O ou N, un groupe nitrile ou le groupe NO₂;
Z représente un groupe acide carboxylique ou un de ses dérivés fonctionnels comme un ester ou un amide, qui peut éventuellement contenir des atomes de O ou N, ou un groupe nitrile; et
Y₁ est identique à Z ou représente un atome d'hydrogène lorsque Z représente un groupe acide carboxylique;
**caractérisé en ce que** l'on ajoute la cétone ou l'aldéhyde en continu ou par portions à une vitesse correspondant exactement à l'avancement de la réaction jusqu'à ce que la concentration du produit (V) soit supérieure à 0,2 mol/l, puis, éventuellement après avoir isolé le produit (V), on transforme de façon connue en soi X₄, X₅ et/ou Y₁ du composé (V) dans le cadre élargi de la signification groupes X₁, X₂ et/ou Y de la formule (I).
